(19) Europäisches Patentamt
European Patent Office
Office européen des brevets

(11) **EP 4 238 601 B1**

(12) **EUROPÄISCHE PATENTSCHRIFT**

(45) Veröffentlichungstag und Bekanntmachung des Hinweises auf die Patenterteilung:
**17.09.2025   Patentblatt 2025/38**

(21) Anmeldenummer: **23157936.8**

(22) Anmeldetag: **22.02.2023**

(51) Internationale Patentklassifikation (IPC):
*A61M 16/00* (2006.01)      *A61M 16/12* (2006.01)
*A61M 16/10* (2006.01)      *G16H 20/00* (2018.01)

(52) Gemeinsame Patentklassifikation (CPC):
(C-Sets verfügbar)
**A61M 16/026; A61M 16/12; G16H 20/40;**
**G16H 40/63; G16H 50/20;** A61M 16/0069;
A61M 16/101; A61M 16/125; A61M 16/203;
A61M 2016/0027; A61M 2016/0036;
A61M 2016/1025; A61M 2202/0208; A61M 2205/18;
A61M 2205/3334;                                      (Forts.)

(54) **VORRICHTUNG ZUR REGELUNG EINES GASFLUSSES**

APPARATUS FOR CONTROLLING GAS FLOW

DISPOSITIF DE RÉGULATION D'UN FLUX DE GAZ

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität:  **02.03.2022   DE 102022104938**

(43) Veröffentlichungstag der Anmeldung:
**06.09.2023   Patentblatt 2023/36**

(73) Patentinhaber: **Löwenstein Medical Technology S.A.**
**2557 Luxembourg (LU)**

(72) Erfinder:
• **Adametz, Benjamin**
  **22609 Hamburg (DE)**
• **Fröhlich, Ralf**
  **22869 Schenefeld (DE)**

(74) Vertreter: **Löwenstein Medical IP**
**Löwenstein Medical Technology GmbH + Co.KG IP Management**
**Kronsaalsweg 40**
**22525 Hamburg (DE)**

(56) Entgegenhaltungen:
**US-A1- 2016 287 824      US-A1- 2021 361 899**
**US-B1- 6 216 690       US-B2- 10 821 259**

(52) Gemeinsame Patentklassifikation (CPC): (Forts.)
A61M 2205/3553

C-Sets
A61M 2202/0208, A61M 2202/0007

**Beschreibung**

Die Erfindung betrifft ein Beatmungsgerät zur Regelung des Gasflusses eines ersten Gases zur Beimischung zu einem zweiten Gas.

In vielen Geräte, insbesondere in medizintechnischen Geräten, ist es vorgesehen, dass zwei Gase miteinander gemischt werden. Zum Teil kann es vorkommen, dass das zweite Gas ein Gasgemisch ist, welches bereits eine bestimmte Konzentration des ersten Gases umfasst. In der Regel wird der Fluss und damit die Menge von zumindest einem der zu mischenden Gase (oder Gasgemische) durch ein Ventil geregelt bzw. erzeugt. In der Regel wird dabei ein Regelkreis oder Steuerkreis verwendet, wonach das Ventil anhand der Konzentration des ersten Gases, beispielsweise Sauerstoff, in dem entstehenden Gemisch gesteuert wird. Die Messung der Sauerstoffkonzentration ist in der Regel aber ungenau und langsam. Dabei ergeben sich zum Beispiel Probleme bei der Versorgung von Patienten mit der richtigen Menge an Sauerstoff zur richtigen Zeit. Auch treten Verzögerungen in der Einstellung der richtigen Konzentration auf.

US 2016/0287824 A1 offenbart ein Beatmungssystem mit einer Steuereinheit zum Steuern eines Sauerstoffventils und eines Luftgebläses basierend auf einem gemessenen Fluss und einem gemessenen Druck eines Gasgemischs aus dem Sauerstoff und der Luft und auf einem gemessenen Fluss des Sauerstoffs.

US 10,821,259 B2 offenbart ein Beatmungsgerät mit einer Steuereinheit zum Steuern eines ersten Gasflusses über ein Gasventil und eines zweiten Gasflusses über ein Gebläse, wobei das Gebläse über eine Rückführung der Motordrehzahl, des Flusses und des Drucks gesteuert wird.

US 2021/0361899 A1 offenbart ein Therapiegerät zum Überwachen einer Blutsauerstoffsättigung ($SpO_2$) und zum Steuern eines einem Patienten zugeführten Sauerstoffanteils ($FdO_2$), wobei der Sauerstoffanteil automatisch eingestellt werden kann, um einen bestimmten Sollwert für die Blutsauerstoffsättigung zu erreichen.

US 6,216,690 B1 offenbart ein Verfahren und ein System zum Steuern der Konzentration eines eingeatmeten Gases im Betrieb eines Anästhesiegeräts mithilfe einer Durchflussprioritätsregelung und einer Konzentrationsprioritätsregelung, zwischen denen automatisch umgeschaltet werden kann.

Aufgabe der vorliegenden Erfindung ist daher die Bereitstellung einer Vorrichtung zur effizienten Regulierung der Gaszusammensetzung.

Diese Aufgabe wird durch die Gegenstände des unabhängigen Anspruchs gelöst.

Die Erfindung betrifft ein Beatmungsgerät nach Anspruch 1.

Das Verfahren zu dessen Ausführung die Steuereinheit des Beatmungsgeräts ausgebildet ist umfasst ferner einen ersten Verfahrensschritt einer Gesamtfluss-Prognose; einen zweiten Verfahrensschritt zur Fluss-Skalierung; einen dritten Verfahrensschritt zur Bestimmung eines prognostizierten Gasfluss-Sollwerts und eines skalierten prognostizierten Gasfluss-Sollwerts.

In dem ersten Verfahrensschritt wird ausgehend von einem eingestellten Druckwert und einer Beatmungssituation ein prognostizierter Gesamtfluss-Sollwert des Gasgemisches der zumindest zwei Gase bestimmt. In manchen Ausführungsformen umfasst der erste Verfahrensschritt des Verfahrens zu dessen Ausführung die Steuereinheit des Beatmungsgeräts ausgebildet ist ein Patientenflussmodell, wobei der eingestellte Druckwert und die Beatmungssituation zumindest teilweise in das Patientenflussmodell eingehen und wobei in eine Berechnung des prognostizierten Gesamtfluss-Sollwertes das Ergebnis des Patientenflussmodells sowie zumindest teilweise die Beatmungssituation eingehen. In manchen Ausführungsformen werden im ersten Verfahrensschritt über ein Patientenmodell, insbesondere in Form eines RC-Ersatzmodells, aus der Beatmungssituation Ausgangswerte für das Patientenflussmodell bestimmt.

In dem zweiten Verfahrensschritt des Verfahrens zu dessen Ausführung die Steuereinheit des Beatmungsgeräts ausgebildet ist wird aus dem prognostizierten Gesamtfluss-Sollwert allein oder aus dem prognostizierten Gesamtfluss-Sollwert zusammen mit dem prognostizierten Gasfluss-Sollwert ein skalierter prognostizierter Gesamtfluss-Sollwert und/oder der skalierte prognostizierte Gasfluss-Sollwert berechnet. In dem zweiten Verfahrensschritt wird aus dem prognostizierten Gasfluss-Sollwert ein skalierter prognostizierter Gesamtfluss-Sollwert und/oder der skalierte prognostizierte Gasfluss-Sollwert berechnet. In manchen Ausführungsformen umfasst der zweite Verfahrensschritt eine Skalierung, wobei für die Inspiration und Exspiration separate Skalierungsfaktoren und/oder Skalierungsfunktionen bestimmt werden und je nach Inspiration oder Exspiration über einen Schalter zwischen den Skalierungsfaktoren und/oder Skalierungsfunktionen umgeschaltet wird. In manchen Ausführungsformen wird zur Bestimmung der Skalierungsfaktoren und/oder der Skalierungsfunktionen zumindest ein Vergleich zwischen einem vorgesehenen Inspirations- oder Exspirationsvolumen und einem tatsächlich applizierten Inspirations- oder Exspirationsvolumen berücksichtigt.

Der dritte Verfahrensschritt des Verfahrens zu dessen Ausführung die Steuereinheit des Beatmungsgeräts ausgebildet ist umfasst eine Berechnung des prognostizierten Gasfluss-Sollwerts aus dem prognostizierten Gesamtfluss-Sollwert. In manchen Ausführungsformen umfasst der dritte Verfahrensschritt die Bestimmung einer mittleren Konzentration mcO2% des ersten Gases im zweiten Gas, wobei bei der Bestimmung der mittleren Konzentration mcO2% des ersten Gases ein Rückatemvolumen miteinbezogen wird. In manchen Ausführungsformen wird im dritten Verfahrensschritt die bestimmte mittlere Konzentration mcO2% des ersten Gases mit in die Bestimmung des prognostizierten Gasfluss-Sollwertes einbezogen.

Die Eingangsgröße für den Feedforward-Anteil zur Bestimmung der Stellgröße zur Steuerung des Gasventils ist der skalierte prognostizierte Gasfluss-Sollwert, optional mit Berücksichtigung des Rückatemvolumens.

In manchen Ausführungsformen umfasst die Eingangsgröße für den Korrekturregler-Anteil zumindest einen Parameter, der eine Abweichung eines durch zumindest einen Flusssensor bestimmten Gasfluss-Istwerts von einem vorgegebenen Gasfluss-Sollwert beschreibt.

In manchen Ausführungsformen wird der Korrekturregler-Anteil der Stellgröße zur Steuerung des Gasventils null, wenn der Gasflusswert dem Gasfluss-Sollwert entspricht.

In manchen Ausführungsformen ist das erste Gas Sauerstoff und das zweite Gas Umgebungsluft oder Druck- oder Pressluft oder ein Gasgemisch aus Umgebungsluft und/oder Druck- oder Pressluft und/oder einem zumindest teilweise zurückgeatmeten Atemgas.

In manchen Ausführungsformen ist die Steuereinheit ausgebildet ist, um das Rückatemvolumen anhand von Messdaten zumindest eines Flusssensors zu berechnen.

In manchen Ausführungsformen wird im ersten Verfahrensschritt aus zumindest einer Beatmungssituation, einem eingestellten Druckwert und zumindest teilweise über ein Patientenflussmodell ein prognostizierter Gesamtfluss-Sollwert berechnet, wobei im zweiten Verfahrensschritt aus dem prognostizierten Gesamtfluss-Sollwert sowie Inspirationsdaten und Exspirationsdaten über eine Skalierung ein skalierter prognostizierter Gesamtfluss-Sollwert bestimmt wird, wobei die Skalierung auf die jeweilige Atemphase angepasst wird, wobei im dritten Verfahrensschritt ein skalierter prognostizierter Gasfluss-Sollwert aus dem skalierten prognostizierten Gesamtfluss-Sollwert und einer mit einem Rückatemvolumen bestimmten mittleren Konzentration $mcO_2\%$ des ersten Gases bestimmt wird, wobei in einem vierten Verfahrensschritt die Eingangsgröße für den Feedforward-Anteil der skalierte prognostizierte Gasfluss-Sollwert ist.

Die Erfindung betrifft ein Beatmungsgerät umfassend zumindest eine Steuereinheit wie zuvor beschrieben.

Die Steuereinheit ist ausgebildet, um ein Verfahren zur Regelung eines Gasflusses zumindest eines ersten Gases zur Beimischung zu zumindest einem zweiten Gas auszuführen, wobei das Verfahren zumindest einen Verfahrensschritt einer Steuerung eines Gasventils umfasst, wobei zumindest eine Stellgröße für die Steuerung des Gasventils aus zumindest einem Korrekturregler-Anteil und zumindest einem Feedforward-Anteil bestimmt wird, wobei die Eingangsgröße des Feedforward-Anteils ein prognostizierter Gasfluss-Sollwert des ersten Gases ist.

In manchen Ausführungsformen umfasst die Steuereinheit eine Berechnungseinheit, die ausgebildet ist, um den prognostizierten Gasfluss-Sollwert eines ersten Gases aus einem prognostizierten Gesamtfluss-Sollwert zu bestimmen.

In manchen Ausführungsformen ist die Berechnungseinheit ausgebildet, um den prognostizierten Gesamtfluss-Sollwert aus einem eingestellten Druckwert und einer Beatmungssituation zu bestimmen.

Die Erfindung betrifft auch ein Beatmungsgerät, wobei das Beatmungsgerät ein Gasventil und eine Steuereinheit umfasst, wobei die Steuereinheit ausgebildet ist, um das zuvor beschriebene Verfahren auszuführen.

In manchen Ausführungsformen umfasst das Beatmungsgerät zumindest einen Flusssensor, wobei die Steuereinheit ausgebildet ist, um das Rückatemvolumen V_Rück anhand der Messdaten zumindest eines Flusssensors zu berechnen.

In manchen Ausführungsformen ist die Steuereinheit ausgebildet, um aus einem Vergleich eines vorgegebenen Gasfluss-Sollwerts und eines durch einen Flusssensor bestimmten Gasflusswerts des Gasflusses des ersten Gases den Korrekturregler-Anteil der Stellgröße zur Steuerung des Gasventils zu bestimmen.

In manchen Ausführungsformen ist die Steuereinheit ausgebildet, um das Gasventil anhand einer Stellgröße zu steuern, wobei die Stellgröße aus einem Feedforward-Anteil und einem Korrekturregler-Anteil besteht und wobei der Feedforward-Anteil als Eingangsgröße den prognostizierten Gasfluss-Sollwert des ersten Gases hat.

In manchen Ausführungsformen umfasst das Beatmungsgerät und/oder die Steuereinheit eine Berechnungseinheit, die ausgebildet ist, um den prognostizierten Gasfluss-Sollwert eines ersten Gases aus einem prognostizierten Gesamtfluss-Sollwert zu bestimmen.

In manchen Ausführungsformen ist die Berechnungseinheit ausgebildet, um aus einem eingestellten Druckwert und einer Beatmungssituation den prognostizierten Gesamtfluss-Sollwert zu bestimmen.

In manchen Ausführungsformen ist die Berechnungseinheit ausgebildet, um ein Patientenflussmodell in die Bestimmung des prognostizierten Gesamtfluss-Sollwertes einzubeziehen.

In manchen Ausführungsformen ist die Berechnungseinheit ausgebildet, um aus dem prognostizierten Gesamtfluss-Sollwert einen skalierten prognostizierten Gesamtfluss-Sollwert und/oder einen skalierten prognostizierten Gasfluss-Sollwert zu berechnen.

In manchen Ausführungsformen ist die Berechnungseinheit ausgebildet, um den prognostizierten Gesamtfluss-Sollwert je nach Atemphase zu skalieren.

In manchen Ausführungsformen ist die Berechnungseinheit ausgebildet, um in die Berechnung des skalierten prognostizierten Gasfluss-Sollwerts eine mittlere Umgebungsluftkonzentration des ersten Gases miteinzubeziehen, wobei die mittlere Umgebungsluftkonzentration des ersten Gases unter anderem über ein Rückatemvolumen V_Rück bestimmt wird.

In manchen Ausführungsformen ist die Berechnungseinheit ausgebildet, um das Rückatemvolumen V_Rück anhand der Messdaten zumindest eines Flusssensors zu berechnen.

In manchen Ausführungsformen ist die Berechnungseinheit ausgebildet, um aus einem Vergleich eines vorgegebenen Gasfluss-Sollwerts und eines durch einen Flusssensor bestimmten Gasflusswerts des Gasflusses des ersten Gases den Korrekturregler-Anteil der Stellgröße zur Steuerung des Gasventils zu bestimmen.

In manchen Ausführungsformen ist die Steuereinheit ausgebildet, um ein Verfahren zur Regelung des Gasflusses zumindest eines ersten Gases zur Beimischung zu zumindest einem zweiten Gas auszuführen, wobei das Verfahren zumindest einen Verfahrensschritt einer Steuerung eines Gasventils umfasst, wobei zumindest eine Stellgröße für die Steuerung des Gasventils aus zumindest einem Korrekturregler-Anteil und zumindest einem Feedforward-Anteil bestimmt wird, wobei

a. in einem ersten Verfahrensschritt aus zumindest einer Beatmungssituation, einem eingestellten Druckwert und zumindest teilweise über ein Patientenflussmodell ein prognostizierter Gesamtfluss-Sollwert berechnet wird;
b. in einem zweiten Verfahrensschritt aus dem Gesamtfluss-Sollwert sowie Inspirationsdaten und Exspirationsdaten über eine Skalierung ein skalierter prognostizierter Gesamtfluss-Sollwert bestimmt wird, wobei die Skalierung auf die jeweilige Atemphase angepasst wird;
c. in einem dritten Verfahrensschritt ein skalierter prognostizierter Gasfluss-Sollwert aus dem skalierten prognostizierten Gesamtfluss-Sollwert und einer mit einem Rückatemvolumen V_Rück bestimmten mittleren Konzentration des ersten Gases mcO2% bestimmt wird;
d. in einem vierten Verfahrensschritt die Eingangsgröße für den Feedforward-Anteil der skalierte prognostizierte Gasfluss-Sollwert aus dem dritten Verfahrensschritt ist.

Die vorgestellte Steuerung, das vorgestellte Verfahren sowie das vorgestellte Beatmungsgerät sind dazu konzipiert, eine Gesamtkonzentration eines spezifischen Gases in einem Gasgemisch zu erreichen. Dabei wird ein erstes Gas einem zweiten Gas bzw. einem Gasgemisch zugemischt, wobei das zweite Gas bzw. Gasgemisch eine Konzentration des ersten Gases beinhaltet. Erreicht wird dies durch eine gezielte Steuerung eines Gasventils, das einen Fluss des ersten Gases erzeugt, das dem zweiten Gas beigemischt wird. In manchen Ausführungsformen ist vorgesehen, dass das Gasgemisch aus dem ersten und dem zweiten Gas zumindest teilweise zurückfließen kann. Es kann dabei vorgesehen sein, die Konzentration des ersten Gases in dem zurückfließenden Gas mit zu berücksichtigen und die Steuerung des Gasventils für den Fluss des ersten Gases entsprechend anzupassen.

Es wird darauf hingewiesen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es wird ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Unter einem Beatmungsgerät ist jedwedes Gerät zu verstehen, das einen Anwender oder Patienten bei der natürlichen Atmung unterstützt, die Beatmung des Anwenders bzw. Patienten, z. B. auch eines Neu- oder Frühgeborenen, übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiLevel-Geräte, Narkose- bzw. Anästhesiegeräte, Atemtherapiegeräte, klinische, außerklinische oder Notfallbeatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen und/oder atmungsbezogenen Parametern eines Lebewesens dienen. Darunter fallen auch Geräte, die medizinische Parameter von Patienten in Kombination mit der Atmung oder ausschließlich die Atmung betreffend erfassen und optional verarbeiten können.

Als Patienteninterface kann, soweit nicht ausdrücklich anders beschrieben, jegliches Peripheriegerät verstanden werden, das zur Interaktion, insbesondere zu Therapie-, Beatmungs- und/oder Diagnosezwecken, eines Beatmungsgeräts mit einem Lebewesen und umgekehrt gedacht ist. Insbesondere kann ein Patienteninterface als eine Maske eines Beatmungsgerätes oder als eine mit dem Beatmungsgerät verbundene Maske verstanden werden. Diese Maske kann eine Full-Face-Maske, also eine Nase und Mund umschließende Maske, oder eine Nasenmaske, also eine nur die Nase umschließende Maske, sein. Auch Trachealtuben bzw. -kanülen und sogenannte Nasenbrillen können als Maske oder Patienteninterface eingesetzt werden. In manchen Fällen kann das Patienteninterface auch ein einfaches Mundstück, beispielsweise ein Rohr, sein, durch das das Lebewesen ausatmet und/oder einatmet.

Die Verbindung zwischen Patienteninterface und Beatmungsgerät kann über eine Vielzahl verschiedener Verbindungs- bzw. Schlauchsysteme erfolgen. Beispielsweise kann ein Leckagesystem vorgesehen sein, bei dem ausgeatmetes Atemgas durch eine gewollte Leckage am Patienteninterface und/oder am Beatmungsschlauch entweichen kann. Auch kann ein Zwei-Schlauch-System vorgesehen sein, bei dem das ausgeatmete Atemgas durch einen Exspirationsschlauch in das Beatmungsgerät zurückgeführt wird, das Ausatemvolumen gegebenenfalls dort gemessen wird und durch das Beatmungsgerät in die Umgebungsluft entweichen kann. Über ein Ventil wird dabei im Wesentlichen verhindert, dass das ausgeatmete Atemgas in den Inspirationsschlauch gelangt. Auch kann vorgesehen sein, dass das Patienteninterface und das Beatmungsgerät über ein Ein-Schlauch-System mit einem Patientenventil verbunden sind, das zum Beispiel über das Beatmungsgerät gesteuert wird. Zumindest während der Exspiration kann das Ventil so geschaltet werden, dass die Ausatemluft direkt aus dem Schlauchsystem in die Umgebungsluft entweichen kann.

In der Beschreibung und den Ansprüchen werden die Begriffe Gas und Gasgemisch synonym verwendet. Insbesondere kann ein Gas auch ein Gasgemisch beschreiben. Insbesondere kann es sich bei dem zweiten Gas um ein Gasgemisch handeln. Das zweite Gas kann dabei beispielsweise die Umgebungsluft sein, die angesaugt wird oder auch Druck- oder Pressluft, die aus Gasflaschen oder einer Versorgungsleitung zugeführt wird. In manchen Ausführungsformen kann das zweite Gas auch eine Mischung aus der angesaugten Umgebungsluft bzw. der Druck- oder Pressluft zusammen mit einem zurückgeatmeten oder zurückfließenden Gasgemisch sein. Das zurückfließende Gasgemisch kann dabei eine Konzentration des ersten Gases aufweisen, die von dem ursprünglich erzeugten Gasgemisch abweicht. In manchen Ausführungsformen handelt es sich bei dem ersten Gas um Sauerstoff und bei dem zweiten Gas um Umgebungsluft und/oder einem zurückfließenden Gasgemisch und/oder eine Mischung daraus.

Es wird ferner darauf hingewiesen, dass unter "prognostizierter Gasfluss-Sollwert" jede Art von prognostiziertem Gasfluss-Sollwert zu verstehen ist, soweit nicht eindeutig anders beschrieben. Insbesondere kann "prognostizierter Gasfluss-Sollwert" neben dem (nicht skalierten) prognostizierten Gasfluss-Sollwert auch einen skalierten prognostizierten Gasfluss-Sollwert umfassen. Ebenso gilt, dass unter "prognostizierter Gesamtfluss-Sollwert" jede Art von prognostiziertem Gesamtfluss-Sollwert zu verstehen ist, soweit nicht eindeutig anders beschrieben. Insbesondere kann "prognostizierter Gesamtfluss-Sollwert" neben dem (nicht skalierten) prognostizierten Gesamtfluss-Sollwert auch einen skalierten prognostizierten Gesamtfluss-Sollwert umfassen.

Die Erfindung folgt dem Grundgedanken, die Eingangsgröße für den Feedforward-Anteil der Stellgröße zu ersetzen. Bei einer klassischen Feedforward-Regelung für den Gasfluss ist zumindest eine Eingangsgröße für den Feedforward-Anteil ein aus einem gemessenen Gesamtfluss berechneter Gasfluss-Sollwert. Dieser aus einem gemessenen Gesamtfluss berechnete Gasfluss-Sollwert wird durch einen prognostizierten Gasfluss-Sollwert ersetzt, der aus einem prognostizierten Gesamtfluss ermittelt wird.

Gemäß dem erfinderischen Verfahren, das zum Beispiel von dem Beatmungsgerät ausgeführt werden kann, wird die Konzentration eines ersten Gases, beispielsweise Sauerstoff, unter anderem durch eine Feedforward-Regelung des Gasflusses des ersten Gases geregelt. Ziel der Feedforward-Regelung ist es, den Gasfluss vorzubestimmen und passend auf den nächsten bzw. aktuellen Atemzug zu regeln. Neben dem Feedforward-Anteil ist auch ein Korrekturregler-Anteil, ähnlich einem Feedback, vorgesehen. Der Korrekturregler-Anteil basiert dabei auf einem Vergleich des tatsächlich gemessenen

Gasflusses mit dem vorgesehenen Wert, um so etwaige Ungenauigkeiten oder Abweichungen zu korrigieren, unabhängig von der Fehlerquelle. Kommt durch den Feedforward-Anteil ein Gasfluss zustande, der genau dem eingestellten Zielwert entspricht, so findet keine Korrekturregelung statt. Durch die Korrekturregelung können beispielsweise auch systematische Fehler der Feedforward-Regelung ausgeglichen werden. Weicht der resultierende Gasfluss aus der Feedforward-Regelung beispielsweise immer um einen bestimmten Prozentsatz von dem vorgegebenen Zielwert ab, so kann dieser durch die Korrekturregelung ausgeglichen werden.

Als Eingangsgröße zur Bestimmung des Feedforward-Anteils dient ein prognostizierter Gasfluss-Sollwert des zu regelnden Gases. Zur Bestimmung des prognostizierten Gasfluss-Sollwerts wird in einem ersten Verfahrensschritt ein Gesamtfluss-Sollwert des Atemgases prognostiziert. In die Prognose wird dabei die aktuelle Beatmungssituation miteinbezogen. Die Beatmungssituation kann dabei zum einen Einstellungen oder Parameter zur Beatmung des Patienten widerspiegeln und zum anderen können auch patientenbezogene Faktoren, zum Beispiel die Physiologie des Patienten, miteinbezogen werden. Neben der aktuellen Beatmungssituation wird in die Berechnung der Gesamtfluss-Prognose auch ein Patientenflussmodell einbezogen. Eingangsgrößen und/oder Faktoren für das Patientenflussmodell sind zumindest ein Teil der aktuellen Beatmungssituation sowie der vorgegebene (Beatmungs-)Druck. Um in das Patientenflussmodell einbezogen zu werden, wird die aktuelle Beatmungssituation beispielsweise über ein RC-Ersatzmodell umgewandelt.

In manchen Ausführungsformen ist vorgesehen, dass durch die Regelung bzw. Steuerung des Gasflusses zum Einstellen der Gaskonzentration des ersten Gases auch durch die Ansteuerung des Gasventils auftretende Ungenauigkeiten kompensiert werden können. Dazu wird in einem weiteren Verfahrensschritt ein Korrekturfaktor bestimmt, der beispielsweise auf die Gesamtfluss-Prognose angewendet wird. Dieser Korrekturfaktor kann beispielsweise auch auf die Atemphase des Patienten - also Inspiration und Exspiration - angepasst sein. Zur Bestimmung des Korrekturfaktors kann vorgesehen sein, dass, optional über mehrere Atemzüge gemittelt, ein Verhältnis einer Volumenvorgabe und/oder eines erwarteten Volumens zu dem tatsächlich gemessenen Volumen erstellt wird. Anhand dieses Verhältnisses wird für die jeweilige Atemphase ein Korrekturfaktor bestimmt, der auf die Gesamtfluss-Prognose anwendet wird.

Es kann vorgesehen sein, dass der prognostizierte Gasfluss-Sollwert des zu regelnden ersten Gases anhand der mit dem Korrekturfaktor verrechneten Gesamtfluss-Prognose bestimmt wird. In manchen Ausführungsformen kann auch vorgesehen sein, dass zunächst der prognostizierte Flusswert des zu regelnden Gases aus der Gesamtfluss-Prognose bestimmt wird und dieser prognostizierte Flusswert dann über den Korrekturfaktor skaliert wird.

In manchen Ausführungsformen ist in einem Verfahrensschritt vorgesehen, dass optional auch ein in das Beatmungsgerät zurückgeatmeter und/oder durch die Ausatmung des Patienten zurückgeschobener Volumenanteil berücksichtigt wird. Dabei wird der zurückgeatmete Volumenanteil bei der Bestimmung des prognostizierten Flusswertes des zu regelnden Gases miteinbezogen. Dieser Verfahrensschritt kann parallel zu, vor oder nach dem Verfahrensschritt der Bestimmung des Korrekturfaktors erfolgen. In manchen Ausführungsformen ist vorgesehen, dass der Verfahrensschritt der Berücksichtigung des Rückatemvolumens nach dem Verfahrensschritt der Bestimmung des Korrekturfaktors erfolgt.

In einer Ausführungsform ist der Verfahrensablauf so vorgesehen, dass in einem ersten Verfahrensschritt eine Gesamtfluss-Prognose durchgeführt wird. In einem zweiten Verfahrensschritt ist dann vorgesehen, dass ein Korrekturfaktor bestimmt wird, der mit der Gesamtfluss-Prognose verrechnet wird. In einem dritten Verfahrensschritt erfolgt eine optionale Bestimmung des Rückatemvolumens. Danach erfolgt die Bestimmung des prognostizierten Gasflusses des zu regelnden Gases, optional unter Einbeziug des Rückatemvolumens. Der prognostizierte Gasfluss des zu regelnden Gases wird dann als Eingangsgröße zur Feedforward-Regelung des Gasflusses genutzt. Zusammen mit einer Korrekturregelung wird ein Steuersignal für die Ventilschaltung erzeugt, wodurch der tatsächliche Gasfluss geregelt bzw. erzeugt wird. Der gemessene tatsächliche Gasfluss ist dabei eine Größe, die zur Korrekturregelung herangezogen wird.

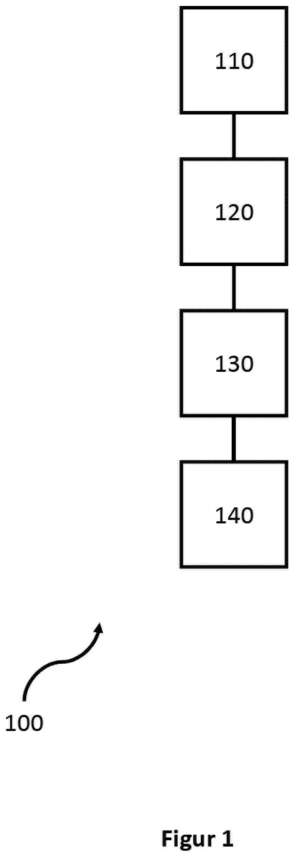

Die Erfindung wird anhand der Figuren 1 bis 6 beispielhaft näher beschrieben.

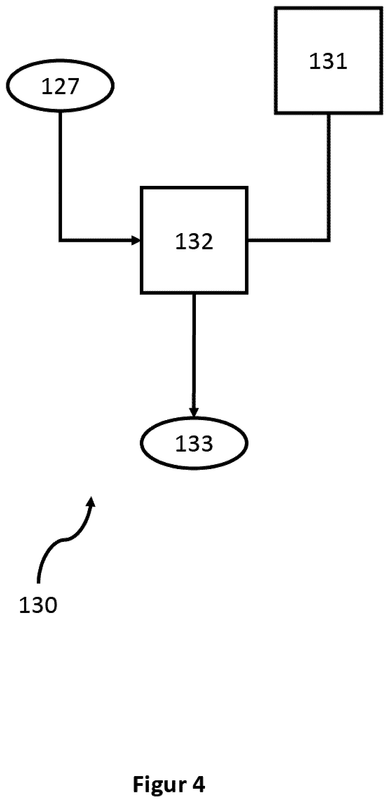

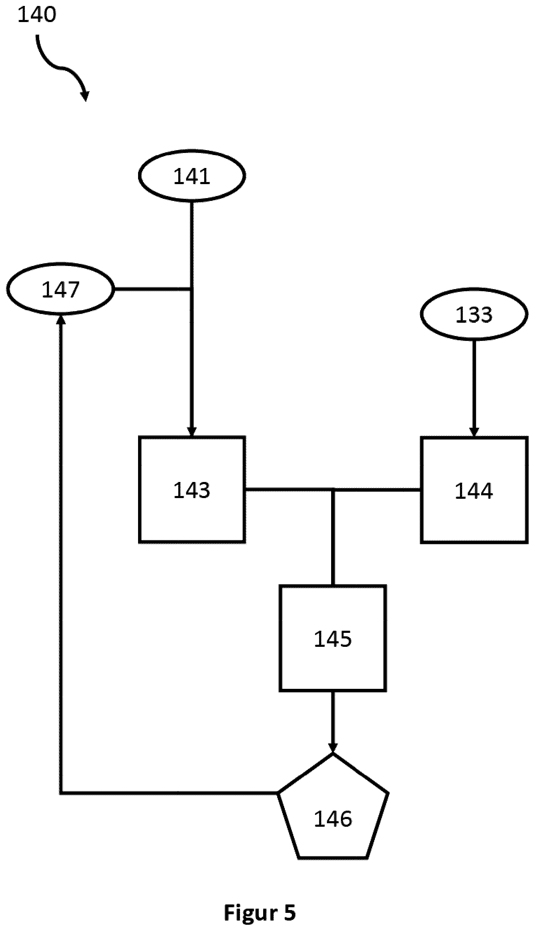

Der Ablauf des Verfahrens 100 zur Regelung des Gasflusses 146 (siehe Figur 5) ist in Figur 1 in einer schematischen Übersicht einer beispielhaften Ausführungsform dargestellt. Das Verfahren 100 umfasst dabei einen Verfahrensschritt 110 der Gesamtfluss-Prognose, einen Verfahrensschritt 120 der Fluss-Skalierung, einen Verfahrensschritt 130 der Bestimmung eines skalierten prognostizierten Gasfluss-Sollwerts 133 (siehe Figur 4) mit optionaler Berücksichtigung des Rückatemvolumens sowie einen Verfahrensschritt 140 der Steuerung des Gasventils. Der Verfahrensschritt 140 der Steuerung des Gasventils umfasst dabei auch die Bestimmung der Stellgröße zur Steuerung des Gasventils aus einem Korrekturregler-Anteil und einem Feedforward-Anteil.

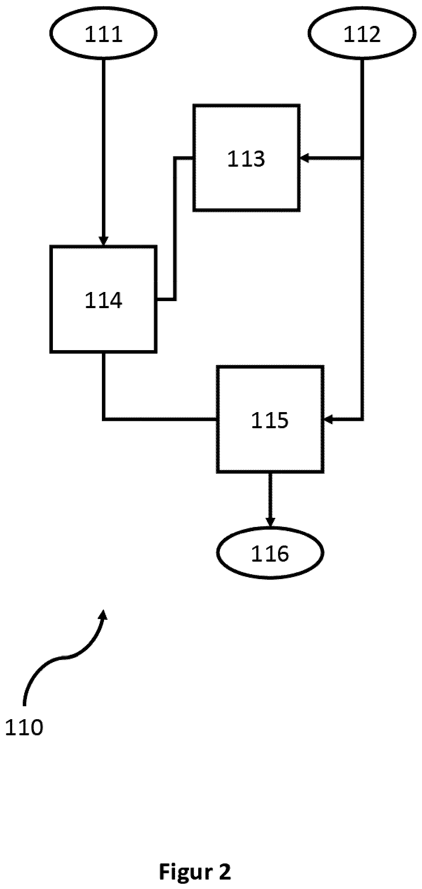

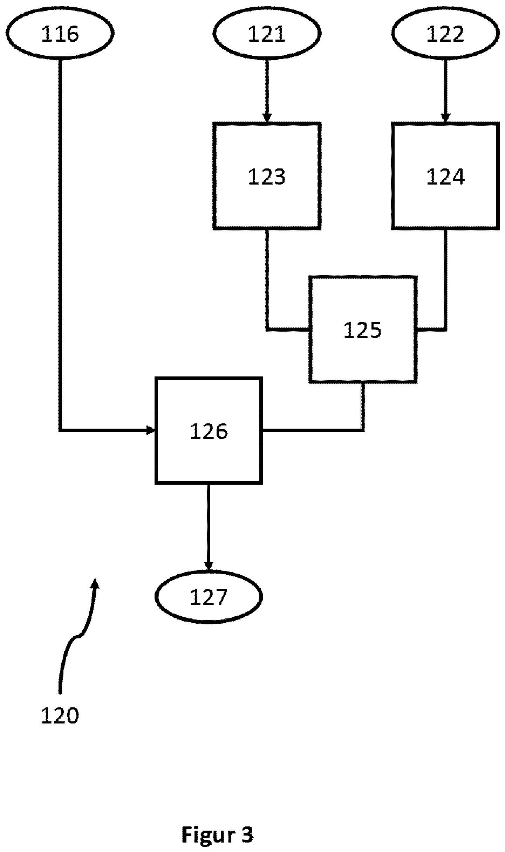

Das Verfahren 100 zur Regelung des Gasflusses 146 beruht auf einer Feedforward-Regelung, wobei die Eingangsgröße des Feedforward-Anteils der Stellgröße zur Steuerung des Gasventils ersetzt wird. Der Vorteil einer Feedforward-Regelung besteht darin, dass die Regelung an einer reinen Steuerung vorbeigeht, sodass eine erhöhte Reaktionszeit, beispielsweise bei Gasflussänderungen, erreicht werden kann. Bei einer klassischen Feedforward-Regelung für den Gasfluss 146 kann zumindest eine Eingangsgröße für den Feedforward-Anteil ein aus einem gemessenen Gesamtfluss berechneter Gasfluss-Sollwert 141 (siehe Figur 5) sein. Dieser aus einem gemessenen Gesamtfluss berechnete Gasfluss-Sollwert 141 wird durch einen prognostizierten Gasfluss-Sollwert ersetzt, der aus einem prognostizierten Gesamtfluss-Sollwert 116 (siehe Figur 2 und Figur 3) ermittelt wird. Die Bestimmung des prognostizierten Gesamtfluss-Sollwerts 116 erfolgt dabei beispielhaft in dem Verfahrensschritt 110 der Gesamtfluss-Prognose. Aus dem prognostizierten Gesamtfluss-Sollwert 116 wird in den Verfahrensschritten 120, 130 der prognostizierte Gasfluss-Sollwert bestimmt und skaliert, um zu einem skalierten prognostizierten Gasfluss-Sollwert 133 zu gelangen. In manchen Ausführungsformen wird dabei zunächst der prognostizierte Gesamtfluss-Sollwert 116 skaliert und aus dem skalierten prognostizierten Gesamtfluss-Sollwert 127 (siehe Figur 3) in Verfahrensschritt 130 der skalierte prognostizierte Gasfluss-Sollwert 133 bestimmt. Alternativ oder ergänzend wird aus dem prognostizierten Gesamtfluss-Sollwert 116 direkt der prognostizierte Gasfluss-Sollwert bestimmt, der dann in Verfahrensschritt 130 entsprechend skaliert wird. Ist die optionale Berücksichtigung des Rückatemvolumens V_Rück vorgesehen, so wird V_Rück bereits vor der Skalierung in die Berechnung des prognostizierten Gasfluss-Sollwertes einbezogen. Optional wird dabei im Verfahrensschritt 130 der Bestimmung des skalierten prognostizierten Gasfluss-Sollwerts 133 auch das Rückatemvolumen V_Rück mit einbezogen, um zum entsprechend angepassten Gasfluss-Sollwert 133 zu gelangen. Der prognostizierte Gasfluss-Sollwert 133 dient in Verfahrensschritt 140 als Eingangsgröße für den Feedforward-Anteil der Stellgröße für die Steuerung des Gasventils.

Der Verfahrensschritt 110 zur Gesamtfluss-Prognose, in dem der prognostizierte Gesamtfluss-Sollwert 116 bestimmt wird, ist in einer beispielhaften Ausführungsform in Figur 2 schematisch dargestellt.

Figur 2 zeigt schematisch einen beispielhaften Ablauf des Verfahrensschrittes 110 zur Gesamtfluss-Prognose. Unter anderem sind die Eingangsgrößen zur Gesamtfluss-Prognose der zur Beatmung vorgegebene Druck 111 sowie die aktuelle Beatmungssituation 112. Die Beatmungssituation 112 kann dabei zum einen Einstellungen oder Parameter zur Beatmung des Patienten widerspiegeln und zum anderen können auch patientenbezogene Faktoren, zum Beispiel die Physiologie des Patienten, mit einbezogen werden. Die Einstellungen oder Parameter zur

Beatmung können etwa Drücke (IPAP, EPAP, PEEP etc.), Flüsse, Volumina, Beatmungszeiten, Beatmungsmodus (CPAP, APAP, BiLevel etc.), Atmungsfrequenzen etc. sein. Patientenbezogene Faktoren wie beispielsweise die Physiologie des Patienten sind beispielsweise Alter, Größe, Gewicht, Geschlecht, Erkrankungen etc.

Eine Möglichkeit, die aktuelle Beatmungssituation 112 zu beschreiben, ist die Modellierung des Patienten über ein RC-Ersatzmodell 113. Neben einem RC-Ersatzmodell können auch andere und/oder weitere Patientenmodelle eingesetzt werden, die den Patienten beispielsweise genauer beschreiben. Die Parametrierung des Patientenflussmodells 114 erfolgt, indem unter anderem die Parameter der vorherigen Atemzüge bewertet werden. Über das RC-Ersatzmodell 113 wird beispielsweise der Verlauf der Patientenparameter aus dem Druck und Fluss geschätzt bzw. bestimmt. Zusammen mit zumindest dem vorgegebenen Druck 111 wird in einem Patientenflussmodell 114 zum Beispiel bestimmt, welcher Gasfluss beim Patienten ankommen soll. Über das beispielhafte RC-Ersatzmodell 113 wird also beispielsweise zunächst der Patient als Ausgangspunkt dargestellt und in dem folgenden Patientenflussmodell 114 wird unter anderem zusammen mit dem eingestellten Beatmungsdruck 111 ein Patientenfluss bestimmt. Beispielhaft werden in dem Zusammenspiel zwischen dem RC-Ersatzmodell 113 und dem Patientenflussmodell 114 die Parameter des vorletzten Atemzuges genutzt, um den letzten Atemzug zu bewerten.

Aus den Berechnungen des Patientenflussmodells 114 sowie der aktuellen Beatmungssituation 112 wird über eine Berechnung 115 der prognostizierte Gesamtfluss 116 berechnet. Die Parameter oder Faktoren der aktuellen Beatmungssituation 112, die in das Patientenflussmodell und direkt in die Berechnung 115 des prognostizierten Gesamtflusses 116 eingehen, können gleich oder unterschiedlich sein. Es kann vorgesehen sein, dass manche Parameter der aktuellen Beatmungssituation 112 sowohl in das Patientenflussmodell 114 als auch direkt in die Berechnung 115 des prognostizierten Gesamtflusses 116 eingehen. Manche Parameter der aktuellen Beatmungssituation 112 können auch nur in das Patientenflussmodell 114 oder die Berechnung 115 direkt eingehen.

Der prognostizierte Gesamtfluss-Sollwert 116 gibt dabei den Gesamtfluss wieder, der zum oder vom Patienten gefördert wird, beispielsweise also den Fluss der angesaugten Umgebungsluft plus den Gasfluss 146.

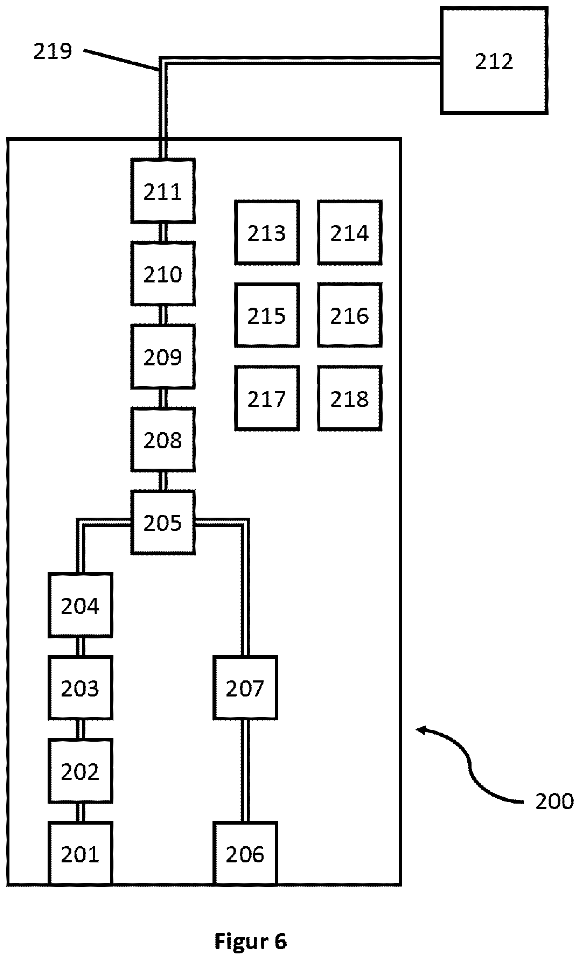

Der Verfahrensschritt 120 der Fluss-Skalierung ist in einer beispielhaften Ausführungsform in Figur 3 schematisch dargestellt. In einem Skalierungsschritt 126 wird der prognostizierte Gesamtfluss-Sollwert 116 skaliert. Als weitere Eingangsgröße für die Skalierung 126 werden dazu Inspirationsdaten 121 und Exspirationsdaten 122 des Patienten 212 (siehe Figur 6) herangezogen. In den Kalkulationsschritten 123, 124, die jeweils einzeln für die Inspiration und die Exspiration durchgeführt werden, wird ein Skalierungsfaktor bzw. eine Skalierungsfunktion bestimmt, mit der der prognostizierte Gesamtfluss-Sollwert 116 skaliert wird. Über einen Schalter 125 wird dabei je nach Atemphase zwischen Skalierung für die Inspiration bzw. Exspiration umgeschaltet.

Die Inspirationsdaten 121 bzw. Exspirationsdaten 122 sind beispielsweise berechnete Werte, die zumindest ein Verhältnis zwischen einem vorgesehenen Inspirations- bzw. Exspirationsvolumen und dem tatsächlich applizierten Inspirations- bzw. Exspirationsvolumen berücksichtigen. Optional wird dieses Verhältnis über mehrere Atemzüge gemittelt und ggf. gewichtet. Eine Gewichtung kann beispielsweise anhand der Aktualität der Atemzüge erfolgen. Beispielsweise werden aktuellere Atemzüge höher gewichtet als Atemzüge, die weiter in der Vergangenheit liegen. In manchen Ausführungsformen kann die Bestimmung des Skalierungsfaktors bzw. der Skalierungsfunktion auch weitere Daten und Einflüsse berücksichtigen.

In Figur 4 ist eine beispielhafte Ausführungsform des Verfahrensschritt 130 zur Bestimmung des skalierten prognostizierten Gasfluss-Sollwertes 133 schematisch dargestellt. Der Verfahrensschritt 130 umfasst in der beispielhaften Ausführungsform auch die optionale Berücksichtigung des Rückatemvolumens V_Rück. Das Rückatemvolumen V_Rück wird beispielhaft über die Bestimmung 131 einer mittleren Konzentration mcO2% des ersten Gases (beispielsweise Sauerstoff) im zweiten Gas oder Gasgemisch in die Berechnung 132 des skalierten prognostizierten Gasfluss-Sollwerts 133 einbezogen. Die mittlere Konzentration mcO2% des ersten Gases im zweiten Gas lässt sich am Beispiel Sauerstoff vereinfacht über die Formel

$$mcO2\% = \frac{(cO2R\ddot{u}ck \cdot V_{R\ddot{u}ck}) + ((V_{Ges} - V_{R\ddot{u}ck}) \cdot 21\%)}{V_{Ges}}$$

berechnen, wobei cO2Rück die Sauerstoffkonzentration in dem Rückatemvolumen V_Rück ist und V_Ges das gesamte Atemvolumen des letzten Atemzuges. Hierbei ist anzumerken, dass das zweite Gas beispielsweise ein Gasgemisch ist, das eine gewisse Konzentration des ersten Gases umfassen kann. Als Beispiel kann hier das Gasgemisch (zweites Gas) Atemgas angeführt werden, das eine gewisse Konzentration an Sauerstoff (erstes Gas) beinhaltet. Die mittlere Konzentration mcO2% berücksichtigt dabei sowohl die frisch angesaugte Umgebungsluft als auch das zurückgeatmete Atemgas. Ziel der Betrachtung des Rückatemvolumens V_Rück ist, dass eine Gesamtkonzentration des ersten Gases in dem zweiten Gas eingestellt werden kann.

Dabei sind die Konzentrationen des ersten Gases in dem ersten Gas (100 %) und dem zweiten Gas zumindest annähernd bekannt. Die Rückatmung, also das Zurückfließen des erzeugten Gasgemisches aus dem ersten und dem zweiten Gas, verändert dabei die Konzentration des ersten Gases in dem Gasgemisch, zu dem das erste Gas zugemischt werden soll. Anstelle der gemessenen Sauerststoffkonzentration cO2Rück kann auch die Zielkonzentration des ersten Gases (hier: Sauerstoff) im zweiten Gas (hier: dem Atemgas) verwendet werden. Die angegebenen 21 % beziehen sich auf den Sauerstoffgehalt der Umgebungsluft. Es kann alternativ oder ergänzend auch vorgesehen sein, dass der Sauerstoffgehalt der angesaugten Umgebungsluft gemessen wird und der gemessene Wert anstatt der angegebenen 21 % ersetzt wird. Ist vorgesehen, dass über das vorgeschlagene Verfahren der Fluss eines anderen Gases als Sauerstoff geregelt werden soll, so sind die Werte entsprechend anzupassen. Ein ergänzender oder alternativer Ansatz kann auch sein, dass nicht eine konstante, sondern eine hohe und dann abfallende Konzentration des ersten Gases als Zielgröße verwendet wird. So kann beispielsweise im Rückatemvolumen die Konzentration kleiner sein und im nächsten Atemzug das erste Gas entsprechend der Zielkonzentration zugemischt werden.

In die Berechnung 132 des skalierten prognostizierten Gasfluss-Sollwerts 133 wird beispielsweise neben der mittleren Umgebungsluftkonzentration mcO2% und dem skalierten prognostizierten Gesamtfluss-Sollwert 127 zumindest auch die Zielkonzentration des ersten Gases in dem Atemgas einbezogen.

Der Verfahrensschritt 140 zur Steuerung 145 des Gasventils ist in einer beispielhaften Ausführungsform in Figur 5 schematisch dargestellt. Die beispielhafte Ausführungsform in Figur 5 umfasst auch die Bestimmung der Stellgröße zur Steuerung 145 des Gasventils 203 (siehe Figur 6) aus dem Korrekturregler-Anteil 143 und dem Feedforward-Anteil 144. Eine der Eingangsgrößen für den Feedforward-Anteil 144 ist dabei der skalierte prognostizierte Gasfluss-Sollwert 133, der beispielhaft im Verfahrensschritt 130 bestimmt wird.

Für den Korrekturregler-Anteil 143 wird der tatsächliche Gasfluss-Istwert 147 mit dem vorgegebenen Gasfluss-Sollwert 141 abgeglichen. Entspricht der tatsächliche Gasfluss-Istwert 147 dem vorgegebenen Gasfluss-Sollwert 141, so wird der Korrekturregler-Anteil 143 null. Es ist also keine Abweichung festzustellen. Der Korrekturregler-Anteil 143 ist beispielsweise unabhängig von der Ursache einer möglichen Abweichung und gibt lediglich an, ob und um wie viel der eingestellte Gasfluss-Sollwert 141 und der tatsächliche Gasfluss-Istwert 147 voneinander abweichen. In manchen Ausführungsformen kann ergänzend vorgesehen sein, dass eine Fehleranalyse durchgeführt wird und auch die Ursache der Abweichung mit in den Korrekturregler-Anteil 143 einbezogen wird. Alternativ oder ergänzend kann vorgesehen sein, dass bei Abweichungen zwischen dem tatsächlichen Gasfluss-Istwert 147 und dem eingestellten Gasfluss-Sollwert 141 über den Korrekturregler-Anteil 143 eine ursachenunabhängige Korrektur vorgenommen wird und gleichzeitig eine Fehleranalyse durchgeführt wird und diese in den Feedforward-Anteil 144 einfließt.

Der Feedforward-Anteil 144 ist so ausgelegt, dass etwaige Störungen und/oder Ungenauigkeiten der Ventilansteuerung bereits vorab einbezogen werden können. Bei einer regulären Feedforward-Regelung kann die Bestimmung des Feedforward-Anteils 144 anhand des vorgegebenen Gasfluss-Sollwerts 141, der wiederum aus einem gemessenen Gesamtfluss bestimmt wird, erfolgen. In dem beschriebenen Verfahren basiert der Feedforward-Anteil 144 hingegen auf einem prognostizierten Gasfluss-Sollwert, zum Beispiel einem skalierten prognostizierten Gasfluss-Sollwert 133. Dadurch kann beispielsweise die Reaktionszeit minimiert und/oder die Regelgüte verbessert werden. Der Feedforward-Anteil 144 wird dabei entsprechend dem bekannten Stand der Technik bestimmt. Der Feedforward-Anteil 144 kann beispielswiese über ein Modell, etwa ein inverses Systemmodel, des zu steuernden Ventils oder einer Kennlinie des Ventils bestimmt werden. In manchen Ausführungsformen kann auch eine Mischung aus den Formen und/oder können andere Modelle und/oder Kennlinien zur Bestimmung des Feedforward-Anteils 144 vorgesehen sein.

Eine beispielhafte Ausführungsform eines Beatmungsgerätes 200 ist schematisch und stark vereinfacht in Figur 6 dargestellt. Das Beatmungsgerät 200 ist, insbesondere durch die Steuereinheit 214 und die Berechnungseinheit 213 ausgebildet, um ein Gasventil 203 über eine Feedforward-Regelung zu steuern.

Das Beatmungsgerät 200 ist ausgebildet, um die Atmung des Patienten 212 zu überwachen und zumindest teilweise zu analysieren. Beispielsweise ist das Beatmungsgerät 200 ausgebildet, um die Atemphasen (Inspiration, Exspiration) zu erkennen. Auch kann das Beatmungsgerät 200 ausgebildet sein, um verschiedene Atemsituationen wie etwa Apnoen, Schnarchen, Atemaussetzer, unregelmäßige Atmung, schwache Atmung etc. zu erkennen. Es kann vorgesehen sein, dass das Beatmungsgerät 200 ausgebildet ist, um zumindest teilweise bestimmte Beatmungsparameter wie Druck und/oder Fluss und/oder Volumen und/oder Frequenz zur Unterstützung und/oder Vorgabe der Atmung automatisch einzustellen. Das Beatmungsgerät 200 ist ausgebildet, um den Patienten 212 zumindest phasenweise bei der Atmung zu unterstützen und/oder die Atmung des Patienten 212 vorzugeben.

Das Beatmungsgerät 200 umfasst zumindest zwei Gasquellen 201, 206. Die Gasquellen 201, 206 sind ausgebildet, um dem Beatmungsgerät 200 jeweils zumindest ein Gas bzw. Gasgemisch zur Verfügung zu stellen.

In der gezeigten beispielhaften Ausführungsform stellt die Gasquelle 201 eine Sauerstoffquelle dar. Beispielhaft umfasst die Sauerstoffquelle 201 zumindest einen Anschluss für einen Druckgasanschluss, etwa eine Sauerstoffflasche. Es kann alternativ oder ergänzend auch vorgesehen sein, dass die Sauerstoffquelle 201 einen in das Beatmungsgerät 200 integrierten oder verbundenen Sauerstoffkonzentrator umfasst. Ist eine Druckgasquelle, zum Beispiel eine Sauerstoffflasche, mit der Sauerstoffquelle 201 des Beatmungsgeräts 200 verbunden, so kann vorgesehen sein, dass ein Druckminderer zwischen der Druckgasquelle und dem Beatmungsgerät 200 angeordnet ist. Alternativ oder ergänzend kann auch ein Druckminderer von der Sauerstoffquelle 201 in bzw. an dem Beatmungsgerät 200 angeordnet sein. Es kann zudem optional vorgesehen sein, dass die Sauerstoffquelle 201 einen Filter umfasst.

Mit der Sauerstoffquelle 201 ist beispielhaft ein Drucksensor 202 pneumatisch verbunden, der ausgebildet ist, um den Druck des gelieferten Sauerstoffs zu messen. In manchen Ausführungsformen ist das Beatmungsgerät 200 ausgebildet ist, um anhand des gemessenen Drucks des Drucksensors 202 einen Rückschluss auf den Füllstand der Sauerstoffquelle 201 bzw. einer damit verbundenen Druckgasquelle zu ziehen. In manchen Ausführungsformen wird bei einem niedrigen Füllstand eine Warnung oder ein Alarm über die Benutzerschnittstelle 218 ausgegeben.

Auf den Drucksensor 202 folgt ein steuerbares Ventil 203, über das der Sauerstofffluss 106 von der Sauerstoffquelle 201 zum Mischbereich 205 geregelt wird. Die Steuerung des Ventils 203 erfolgt beispielhaft über die Steuereinheit 214 anhand des vorstehend beschriebenen Verfahrens.

Auf das Ventil 203 folgend ist ein Flusssensor 204 angeordnet, der ausgebildet ist, um den Fluss des Sauerstoffstroms zu messen.

Die zumindest zweite Gasquelle 206 ist in der gezeigten Ausführungsform beispielhaft als ein Ansaugbereich 206 für Umgebungsluft ausgebildet. Der Ansaugbereich 206 ist optional mit einem Filter ausgestattet, der die Umgebungsluft filtert. Beispielsweise werden so Pathogene und andere Verunreinigungen aus der Umgebungsluft zumindest teilweise herausgefiltert. In manchen Ausführungsformen kann der Ansaugbereich 206 eine Gasfördereinheit umfassen, die ausgebildet ist, um Umgebungsluft anzusaugen. In der beispielhaft dargestellten Ausführungsform in Figur 5 wird die Umgebungsluft durch das Gebläse 208 über den Ansaugbereich 206 angesaugt. Alternativ oder ergänzend zu dem Ansaugbereich 206 kann das Beatmungsgerät 200 eine Verbindung zu einer Druckgasquelle, etwa einer Druckluftflasche, aufweisen. Auf die Gasquelle 206 bzw. den

Ansaugbereich 206 folgend ist ein Flusssensor 207 angeordnet, der ausgebildet ist, um den Gasfluss aus dem Ansaugbereich 206 in Richtung Mischbereich 205 zu messen.

Das Beatmungsgerät 200 umfasst einen Mischbereich 205, in dem die Gasflüsse aus den Gasquellen 201, 206 zusammenkommen und zumindest teilweise durchmischt werden. In manchen Ausführungsformen ist der Mischbereich 205 als eine Mischkammer ausgebildet, die ein Volumen aufweist und optional über bestimmte Strukturen verfügt, die eine Vermischung der beiden Gase verbessern. Es kann alternativ oder ergänzend eine aktive Vermischung, beispielsweise durch Rotoren und/oder Verwirbler, vorgesehen sein. In manchen Ausführungsformen wird der Mischbereich 205 durch eine einfache Zusammenführung der beiden Gaswege von den Gasquellen 201, 206 umgesetzt. Beispielsweise kann dazu ein Y-Stück vorgesehen sein.

Auf den Mischbereich 205 folgend ist ein steuerbares Gebläse 208 angeordnet, das ausgebildet ist, um das Gasgemisch, beispielsweise als Atemgas, aus dem Mischbereich 205 zu fördern. Dabei ist zu beachten, dass in manchen Situationen das Ventil 203 geschlossen ist und kein Gas aus der Gasquelle 201 in den Mischbereich 205 strömt, sodass das Gasgemisch bzw. das Atemgas aus dem Mischbereich 205 unter Umständen ausschließlich aus dem angesaugten Gas oder Gasgemisch aus der Gasquelle oder dem Ansaugbereich 206 besteht. In manchen Ausführungsformen ist das Gebläse 208 auch ausgebildet, um Umgebungsluft über den Ansaugbereich 206 anzusaugen. Das Gebläse 208 ist ausgebildet, um einen einstellbaren Druck und/oder Fluss an Atemgas - gefördert aus dem Mischbereich 205 - bereitzustellen.

Über den Sauerstoffsensor 209 wird der Sauerstoffgehalt des geförderten Atemgases gemessen. Der gemessene Sauerstoffgehalt kann beispielsweise in die Steuerung des Ventils 203 mit einbezogen werden, beispielsweise um Abweichungen gegenüber einem Sollwert korrigieren zu können. Beispielsweise kann die Umgebungsluft eine andere Sauerstoffkonzentration als die angenommenen 21 % aufweisen, sodass zwar der richtige Sauerstofffluss 116 eingestellt wird, aber die Sauerstoffkonzentration im Atemgas dennoch nicht dem Sollwert entspricht.

Beispielhaft auf den Sauerstoffsensor 209 folgend ist ein Drucksensor 210 angeordnet, der ausgebildet ist, um den Atemgasdruck der Atemgasleitung hinter dem Gebläse 208 zu erfassen. Beispielhaft auf den Drucksensor 210 folgend ist ein Flusssensor 211 angeordnet, der ausgebildet ist, um den Atemgasfluss zu erfassen bzw. zu messen.

Das Beatmungsgerät 200 ist beispielhaft über ein Schlauchsystem 219 und ein Patienteninterface (nicht dargestellt) mit dem Patienten 212 verbunden. Als Schlauchsystem 219 kann beispielsweise ein Leckagesystem oder ein Zwei-Schlauch-System oder ein Ein-Schlauch-Ventil-System genutzt werden. Bei einem Leckagesystem weist das Schlauchsystem und/oder das Patienteninterface eine gewollte Leckage auf, durch die das vom Patienten 212 ausgeatmete Atemgas zumindest teilweise entweichen kann. Dabei wird in manchen Ausführungsformen ein Teil des ausgeatmeten Atemgases auch in Richtung des Beatmungsgerätes 200 geleitet. Es kann dabei vorgesehen sein, dass das Volumen des Patienteninterface und des Schlauchsystems so berücksichtigt werden, dass ein Eindringen des ausgeatmeten Atemgases in das Beatmungsgerät 200 im Wesentlichen verhindert werden kann. In manchen Ausführungsformen des Beatmungsgerätes 200 ist im Bereich des Anschlusses des Schlauchsystems 219 an das Beatmungsgerät 200 ein zusätzliches Ventil, beispielsweise ein Rückschlagventil, vorgesehen, das eine Rückatmung in das Beatmungsgerät 200 im Wesentlichen verhindert. Bei einem Ein-Schlauch-Ventil-System ist beispielsweise vorgesehen, dass ein patientennahes Ventil durch das Beatmungsgerät 200 so gesteuert wird, dass zumindest zeitweise während der Exspiration des Patienten die Ausatemluft direkt durch das Ventil in die Umgebung entweichen kann.

Zur Verwendung eines Zwei-Schlauch-Systems verfügt das Beatmungsgerät 200 über separate Inspirations- und Exspirationszweige (nicht dargestellt). Über den Inspirationszweig wird während der Inspiration des Patienten 212 Atemgas zum Patienten 212 gefördert. Über den Exspirationszweig wird während der Exspiration des Patienten 212 das ausgeatmete Atemgas durch das Beatmungsgerät 200 und in die Umgebung geleitet. In manchen Ausführungsformen wird das ausgeatmete Atemgas über einen Filter im Exspirationszweig gefiltert. Bei der Verwendung eines Zwei-Schlauch-Systems ist beispielsweise im Schlauchsystem 219 ein Ventil angeordnet, das während der Exspirationsphase den Inspirationszweig verschließt, sodass kein ausgeatmetes Atemgas in den Inspirationszweig dringen kann. Das ausgeatmete Atemgas wird dabei durch einen zweiten Schlauch zu einem Exspirationszweig in das Beatmungsgerät 200 zurückgeführt und durch diesen beispielsweise in die Umgebung geleitet. Beispielsweise durch Ventilverzögerungen kann es vorkommen, dass ein geringer Teil des Atemgases innerhalb des Inspirationszweiges des Beatmungsgerätes 200 zurückgedrängt wird. Im Wesentlichen wird dabei aber verhindert, dass ausgeatmetes Atemgas den Inspirationszweig des Beatmungsgerätes 200 erreicht.

Beispielhaft umfasst das Beatmungsgerät 200 zumindest eine Berechnungseinheit 213, eine Steuereinheit 214, eine Erfassungseinheit 215, eine Überwachungseinheit 216, eine Speichereinheit 217 und eine Benutzerschnittstelle 218.

Die Berechnungseinheit 213 ist ausgebildet, um die Stellgröße zur Steuerung des Gasventils 203 und/oder den Feedforward-Anteil 144 und/oder den Korrekturregler-Anteil 143 und/oder die Eingangsgröße, z. B. einen prognostizierten Gasfluss-Sollwert, für den Feedforward-Anteil 144 zu berechnen.

Die Berechnungseinheit 213 ist beispielhaft ausgebildet, um das Verfahren 100 zumindest teilweise auszuführen. In manchen Ausführungsformen ist die Berechnungseinheit 213 ausgebildet, um die Berechnungsschritte des Verfahrens 100 auszuführen, und die Steuereinheit 214 ausgebildet, um die darauf basierende Steuerung des Gasventils 203 auszuführen. Die Berechnungseinheit 213 ist ausgebildet, um in einem ersten Verfahrensschritt 110 aus der aktuellen Beatmungssituation 112 und einem eingestellten Beatmungsdruck 111 (PressureSetpoint) einen Gesamtfluss-Sollwert 116 vorherzusagen oder zu prognostizieren. In einem weiteren Verfahrensschritt 120 berechnet die Berechnungseinheit 213 aus dem prognostizierten Gesamtfluss-Sollwert 116 sowie Inspirationsdaten 121 und Exspirationsdaten 122 einen skalierten Gesamtfluss-Sollwert 127. Aus dem skalierten Gesamtfluss-Sollwert 127 und unter optionaler Einbeziehung des Rückatemvolumens V_Rück bestimmt die Berechnungseinheit in einem weiteren Verfahrensschritt 130 einen skalierten prognostizierten Gasfluss-Sollwert 133.

In manchen Ausführungsformen kann vorgesehen sein, dass die Berechnungseinheit 213 zunächst im Verfahrensschritt 130 aus dem prognostizierten Gesamtfluss-Sollwert 116 einen prognostizierten Gasfluss-Sollwert, optional unter Einbeziehung des Rückatemvolumens V_Rück, berechnet. Dieser prognostizierte Gasfluss-Sollwert dient dann an der Stelle des Gesamtfluss-Sollwerts 116 im Verfahrensschritt 120 als Eingangswert für die Bestimmung eines skalierten Gasfluss-Sollwerts 133 (anstatt eines skalierten Gesamtfluss-Sollwerts 127). Der Verfahrensschritt 120 und der Verfahrensschritt 130 können also vertauschbar sein, wobei je nach Reihenfolge zunächst ein skalierter Gesamtfluss-Sollwert 127 und dann der skalierte Gasfluss-Sollwert 133 bestimmt wird oder zunächst aus dem prognostizierten Gesamtfluss-Sollwert 116 ein prognostizierter Gasfluss-Sollwert berechnet wird, der dann skaliert wird, um den skalierten Gasfluss-Sollwert 133 zu erhalten.

Der skalierte prognostizierte Gasfluss-Sollwert 133 dient im Verfahrensschritt 140 dann als Eingangsgröße des Feedforward-Anteils 144 der Stellgröße zur Steuerung 145 des Gasventils 203. Die Stellgröße kann beispielsweise durch die Steuereinheit 214 und/oder durch die Berechnungseinheit 213 bestimmt werden. Die Steuereinheit 214 ist dabei ausgebildet, um anhand der aus dem Feedforward-Anteil 144 und einem Korrekturregler-Anteil 143 bestimmten Stellgröße das Gasventil 203 zu steuern.

In manchen Ausführungsformen kann vorgesehen sein, dass in der Berechnungseinheit 213 die Verfahrensschritte 110, 120, 130 sowie die Bestimmung des Feedforward-Anteils 144 über eine einzelne Berechnung dargestellt wird, wobei die jeweiligen Eingangsgrößen als Variablen eingesetzt sind. In manchen Ausführungsformen kann vorgesehen sein, dass die eine Berechnung auch die Bestimmung der Stellgröße zur Steuerung 145 des Gasventils 203 umfasst. Die in Figur 1 bis 5 dargestellten Verfahrensschritte können also zumindest teilweise in einer einzelnen Berechnung durchgeführt werden, wobei die einzelnen Verfahrensschritte beispielsweise einzelnen Teile einer Gleichung repräsentieren.

Der Korrekturregler-Anteil 143 basiert auf einem Vergleich eines eingestellten Gasfluss-Sollwerts 141 und dem tatsächlichen, gemessenen Gasfluss-Istwert 147. Der Gasfluss-Istwert 147 wird beispielsweise durch den Flusssensor 204 gemessen und durch die Erfassungseinheit 215 erfasst.

Die Steuereinheit 214 ist ausgebildet, um zumindest das Ventil 203 zu steuern. In manchen Ausführungsformen ist die Steuereinheit 214 auch ausgebildet, um das Gebläse 208 zu steuern. Es kann zudem vorgesehen sein, dass die Steuereinheit 214 ausgebildet ist, um das gesamte Beatmungsgerät 200 zu steuern. In manchen Ausführungsformen kann alternativ oder ergänzend vorgesehen sein, dass für verschiedene Funktionen und/oder Module und/oder Komponenten separate Steuereinheiten vorhanden sind.

Es kann in manchen Ausführungsformen vorgesehen sein, dass die Berechnungseinheit 213 zumindest teilweise in die Steuereinheit 214 integriert ist und/oder die Steuereinheit 214 die Berechnungseinheit 213 umfasst.

Die Erfassungseinheit 215 ist ausgebildet, um die von den Sensoren gemessenen Werte zu erfassen und gegebenenfalls aufzubereiten. In manchen Ausführungsformen ist die Erfassungseinheit 215 ausgebildet, um von den Sensoren erzeugte Signale, beispielsweise in Form von Spannung und/oder Stromstärke und/oder Frequenz, zu empfangen und/oder aufzuzeichnen. Es kann alternativ oder ergänzend vorgesehen sein, dass die Erfassungseinheit 215 ausgebildet ist, um die Signale der Sensoren in Werte umzuwandeln.

Die Überwachungseinheit 216 ist ausgebildet, um (technische) Probleme des Beatmungsgerätes 200 zu erfassen. Technische Probleme können zum Beispiel ein niedriger Akkustand, Fehler in der Elektronik, ein defekter Akku, ein defektes Bauteil und/oder Modul, ein Stromausfall, ein nicht korrekt funktionierendes Zubehörteil, ein nicht plausibler Messwert oder ein Verlassen eines erlaubten Temperaturbereichs sein. Die Überwachungseinheit 216 kann ferner ausgebildet sein, um bei einem erkannten technischen Problem einen Alarm und/oder eine Meldung zu erzeugen. In manchen Ausführungsformen kann auch vorgesehen sein, dass die Überwachungseinheit 216 ausgebildet ist, um einen niedrigen Füllstand und/oder eine unzureichende Versorgung mit einem Betriebsmittel, etwa einem Gas, zu erkennen. Beispielsweise kann vorgesehen sein, dass die Überwachungseinheit 216 ausgebildet ist, um anhand eines Drucks der Gasquelle 201 des ersten Gases zu erkennen, ob die Versorgung nachlässt und/oder nicht ausreichend ist.

In der Speichereinheit 217 werden die (Zwischen-)Ergebnisse der Berechnungseinheit 213 sowie die durch die Erfassungseinheit 215 erfassten Messwerte gespeichert und/oder zwischengespeichert. Eine Zwischenspeicherung bedeutet dabei, dass die Daten nach einer gewissen Zeitspanne und/oder einer (automatisch) ausgeführten Aktion und/oder einem Ereignis automatisch gelöscht oder zum Überschreiben freigegeben werden.

Über die Benutzerschnittstelle 218, beispielsweise eine Einheit umfassend Eingabeelemente und Ausgabeelemente, können Daten und/oder Informationen eingegeben und ausgegeben werden. Über die Eingabeelemente können beispielsweise Einstellungen zur Beatmung und zur Konfiguration des Beatmungsgerätes 200 vorgenommen werden. Das Ausgabeelement, beispielsweise ein Display, ist ausgebildet, um Informationen und Daten zur aktuellen Beatmung und zu Einstellungen darzustellen. Über die Benutzerschnittstelle 218 können zudem Alarme und Warnungen bezüglich der Funktionsfähigkeit des Gerätes und auch zur Beatmung des Patienten 212 ausgegeben werden. Über die Benutzerschnittstelle 218 können beispielsweise Vorgaben zur Beatmung, z. B. Drücke, Fluss, Volumen, Dauer, Programm, eingegeben werden. Es kann auch ein (initialer) Zielwert für die Sauerstoffkonzentration im Atemgas festgelegt werden. In manchen Ausführungsformen wird der Zielwert der Sauerstoffkonzentration im Atemgas während der Beatmung optional automatisch durch das Beatmungsgerät 200 angepasst. In manchen Ausführungsformen kann zudem vorgesehen sein, dass eine initiale Sauerstoffkonzentration durch einen Benutzer festgelegt wird und das Beatmungsgerät 200 während der Nutzung die Sauerstoffkonzentration automatisch nachstellt. Es kann zum Beispiel auch vorgesehen sein, dass eine Sauerstoffkonzentration zunächst vorgegeben wird und das Beatmungsgerät 200 nur dann die vorgegebene Sauerstoffkonzentration automatisch einstellt, wenn z. B. die Sauerstoffversorgung des Patienten 212 nicht mehr durch den voreingestellten Wert aufrechterhalten werden kann und/oder der Patient 212 in einem schädlichen Maß überversorgt wird. Es kann dazu beispielsweise vorgesehen sein, dass das Beatmungsgerät 200 über ein Pulsoxymeter mit Daten zur Sauerstoffsättigung des Blutes des Patienten 212 versorgt wird.

Über die Benutzerschnittstelle können beispielsweise auch Schwellen- und Grenzwerte, etwa für minimale und maximale Sauerstoffkonzentration, minimaler und maximaler Sauerstofffluss, minimale und maximale Atemgasdrücke etc., eingegeben werden. Die Schwellen- und Grenzwerte können dabei auch als Alarmgrenzen gelten.

In manchen Ausführungsformen umfasst die Benutzerschnittstelle 218 alternativ oder ergänzend eine Schnittstelle zur Verbindung externer Anzeige- und/oder Eingabegeräte wie Bildschirme, Tastaturen und/oder Fernbedienungen.

Es wird darauf hingewiesen, dass in manchen Ausführungsformen neben den gezeigten Komponenten des Beatmungsgerätes 200 weitere Elemente in dem Beatmungsgerät 200 angeordnet sein können und/oder mit diesem verbunden werden können. Beispielsweise kann ein zusätzlicher Atemgasanfeuchter, optional mit Atemgasheizung, vorgesehen sein. Es kann zum Beispiel auch vorgesehen sein, dass ein Vernebler in dem Beatmungsgerät 200 angeordnet ist und/oder mit dem Beatmungsgerät 200 verbunden ist. In manchen Ausführungsformen kann zudem ein Exspirationszweig zusammen mit entsprechenden Ventilen und einer Steuerung vorgesehen sein. Über einen solchen Exspirationszweig wird das vom Patienten 212 ausgeatmete Atemgas in dem Beatmungsgerät 200 zur Umgebungsluft geleitet. Zudem können optional ein oder mehrere Bypass-Ventile in der Pneumatik des Beatmungsgerätes 200 angeordnet sein, beispielsweise um dem Patienten zumindest zeitweise eine Atmung bei fehlender Energieversorgung zu ermöglichen.

Sind zusätzliche Komponenten mit dem Beatmungsgerät 200 verbunden und/oder in dem Beatmungsgerät 200 angeordnet, kann vorgesehen sein, dass diese ebenfalls von der Berechnungseinheit 213 in die Bestimmung eines prognostizierten Gasfluss-Sollwertes mit einbezogen werden. Beispielsweise kann eine Veränderung der Gasströmung durch den Einsatz eines Verneblers und/oder eines Atemgasanfeuchters berücksichtigt werden.

Zudem kann auch vorgesehen sein, dass das Beatmungsgerät 200 über zumindest eine Schnittstelle zur Verbindung mit entfernten Gegenstellen, etwa für die Telemedizin, verfügt.

In manchen Ausführungsformen kann auch vorgesehen sein, dass das Beatmungsgerät 200 als Anästhesiegerät ausgeführt ist. Dazu ist zumindest eine bevorzugt zusätzliche Gasquelle so vorgesehen, dass über diese ein Anästhesiegas in das Atemgas eingeleitet wird. Dazu kann auch vorgesehen sein, dass die Steuerung genutzt wird, um den Fluss des Anästhesiegases zu steuern. Beispielsweise kann vorgesehen sein, dass die Steuerung parallel für die Steuerung des Gasventils 203 für die erste Gasquelle 201 und für die Steuerung eines weiteren Gasventils der Anästhesiegasquelle eingesetzt wird.

Es kann in manchen Ausführungsformen auch vorgesehen sein, dass die Steuereinheit 214 separat, also unabhängig von einem Beatmungsgerät 200, verwendet wird, um ein Gasventil 203 zu steuern. Es kann beispielsweise auch vorgesehen sein, dass die Steuereinheit 214 für die Steuerung eines Gasventils 203 in anderen Zusammenhängen genutzt wird, bei denen eine Vermischung von zumindest zwei Gasen vorgesehen ist. Entsprechendes gilt für das Verfahren. Dabei wird zum Beispiel das Patientenmodell 114 durch ein Modell eines Verbrauchers ersetzt. Die Inspiration kann dabei zum Beispiel als eine Lieferung des Gasgemisches zum Verbraucher angesehen werden, wobei die Exspiration zum Beispiel einem Ausstoßen der verbrauchten oder umgesetzten Gasmischung entsprechen kann.

**Bezugszeichenliste**

| | |
|---|---|
| 100 | Verfahren |
| 110 | Erster Verfahrensschritt |
| 111 | Druck (vorgegeben) |
| 112 | Beatmungssituation |
| 113 | RC-Ersatzmodell |
| 114 | Patientenflussmodell |
| 115 | Berechnung des prognostizierten Gesamtfluss-Sollwerts |
| 116 | Gesamtfluss-Sollwert (prognostiziert) |
| 120 | Zweiter Verfahrensschritt |
| 121 | Inspirationsdaten |
| 122 | Exspirationsdaten |
| 123 | Kalkulationsschritt |
| 124 | Kalkulationsschritt |
| 125 | Schalter |
| 126 | Skalierung |
| 127 | Gesamtfluss-Sollwert (prognostiziert, skaliert) |
| 130 | Dritter Verfahrensschritt |
| 131 | Bestimmung (mcO2%) |
| 132 | Berechnung des prognostizierten Gasfluss-Sollwerts |
| 133 | Gasfluss-Sollwert (prognostiziert) |
| 134 | Gasfluss-Sollwert (prognostiziert, skaliert) |
| 140 | Verfahrensschritt |
| 141 | Gasfluss-Sollwert (vorgegeben) |
| 143 | Korrekturregler-Anteil |
| 144 | Feedforward-Anteil |
| 145 | Steuerung |
| 146 | Gasfluss |
| 147 | Gasfluss-Istwert |
| 200 | Beatmungsgerät |
| 201 | Gasquelle |
| 202 | Drucksensor |
| 203 | Gasventil |
| 204 | Flusssensor |
| 205 | Mischbereich |
| 206 | Gasquelle/Ansaugbereich |
| 207 | Flusssensor |
| 208 | Gebläse |
| 209 | Sauerstoffsensor |
| 210 | Drucksensor |
| 211 | Flusssensor |
| 212 | Patient |
| 213 | Berechnungseinheit |
| 214 | Steuereinheit |
| 215 | Erfassungseinheit |
| 216 | Überwachungseinheit |

217 Speichereinheit
218 Benutzerschnittstelle
219 Schlauchsystem

**Patentansprüche**

1. Beatmungsgerät (200), umfassend ein Gasventil (203) und eine Steuereinheit (214), die ausgebildet ist, um ein Verfahren (100) zur Regelung eines Gasflusses (146) zumindest eines ersten Gases zur Beimischung zu zumindest einem zweiten Gas auszuführen, wobei das Verfahren (100) zumindest einen Verfahrensschritt (140) einer Steuerung (145) des Gasventils (203) umfasst, wobei zumindest eine Stellgröße für die Steuerung (145) des Gasventils (203) aus zumindest einem Korrekturregler-Anteil (143) und zumindest einem Feedforward-Anteil (144) bestimmt wird, wobei die Eingangsgröße des Feedforward-Anteils (144) ein prognostizierter Gasfluss-Sollwert (133, 134) des ersten Gases ist, wobei das Verfahren (100) ferner umfasst:

   einen ersten Verfahrensschritt (110) der Gesamtfluss-Prognose, wobei ausgehend von einem eingestellten Druckwert (111) und einer Beatmungssituation (112) ein prognostizierter Gesamtfluss-Sollwert (116) des Gasgemisches der zumindest zwei Gase bestimmt wird;
   einen zweiten Verfahrensschritt (120) zur Fluss-Skalierung, wobei aus dem prognostizierten Gesamtfluss-Sollwert (116) allein oder aus dem prognostizierten Gesamtfluss-Sollwert (116) zusammen mit dem prognostizierten Gasfluss-Sollwert (133) ein skalierter, prognostizierter Gesamtfluss-Sollwert (127) berechnet wird;
   einen dritten Verfahrensschritt (130) zur Bestimmung eines skalierten, prognostizierten Gasfluss-Sollwerts (134) aus dem skalierten, prognostizierten Gesamtfluss-Sollwert (127);
   wobei die Eingangsgröße für den Feedforward-Anteil (144) zur Bestimmung der Stellgröße zur Steuerung (145) des Gasventils (203) der skalierte, prognostizierte Gasfluss-Sollwert (134) ist.

2. Beatmungsgerät (200) nach Anspruch 1,
   wobei der erste Verfahrensschritt (110) ein Patientenfluss-Modell (114) umfasst, wobei der eingestellte Druckwert (111) und die Beatmungssituation (112) zumindest teilweise in das Patientenfluss-Modell (114) eingehen und wobei in eine Berechnung (115) des prognostizierten Gesamtfluss-Sollwerts (116) das Ergebnis des Patientenfluss-Modells (114) sowie zumindest teilweise die Beatmungssituation (112) eingehen.

3. Beatmungsgerät (200) nach Anspruch 2,
   wobei im ersten Verfahrensschritt (110) über ein Patientenmodell, insbesondere in Form eines RC-Ersatzmodells (113), aus der Beatmungssituation (112) Ausgangswerte für das Patientenfluss-Modell (114) bestimmt werden.

4. Beatmungsgerät (200) nach einem der vorhergehenden Ansprüche,
   wobei der zweite Verfahrensschritt (120) eine Skalierung (126) umfasst, wobei für die Inspiration und Exspiration separate Skalierungsfaktoren und/oder Skalierungsfunktionen bestimmt werden und je nach Inspiration oder Exspiration über einen Schalter (125) zwischen den Skalierungsfaktoren und/oder Skalierungsfunktionen umgeschaltet wird.

5. Beatmungsgerät (200) nach Anspruch 4,
   wobei zur Bestimmung der Skalierungsfaktoren und/oder der Skalierungsfunktionen zumindest ein Vergleich zwischen einem vorgesehenen Inspirations- oder Exspirationsvolumen und einem tatsächlich applizierten Inspirations- oder Exspirationsvolumen berücksichtigt wird.

6. Beatmungsgerät (200) nach einem der vorhergehenden Ansprüche,
   wobei der dritte Verfahrensschritt (130) die Bestimmung einer mittleren Konzentration mcO2% des ersten Gases im zweiten Gas umfasst, wobei bei der Bestimmung der mittleren Konzentration mcO2% des ersten Gases ein Rückatemvolumen miteinbezogen wird.

7. Beatmungsgerät (200) nach einem der vorhergehenden Ansprüche,
   wobei die Eingangsgröße für den Feedforward-Anteil (144) zur Bestimmung der Stellgröße zur Steuerung (145) des Gasventils (203) der skalierte, prognostizierte Gasfluss-Sollwert (134) mit Berücksichtigung eines Rückatemvolumens ist.

8. Beatmungsgerät (200) nach einem der vorhergehenden Ansprüche,
   wobei eine Eingangsgröße für den Korrekturregler-Anteil (143) zumindest einen Parameter umfasst, welcher eine Abweichung eines durch zumindest einen Flusssensor (204) bestimmten Gasfluss-Istwerts (147) von einem vorgegebenen Gasfluss-Sollwert (141) beschreibt.

9. Beatmungsgerät (200) nach Anspruch 8,
   wobei der Korrekturregler-Anteil (143) der Stellgröße zur Steuerung (145) des Gasventils (203) null wird, wenn der Gasfluss-Istwert (147) dem Gasfluss-Sollwert (141) entspricht.

10. Beatmungsgerät (200) nach einem der vorhergehenden Ansprüche,
    wobei das erste Gas Sauerstoff ist und das zweite Gas Umgebungsluft oder Druckluft oder ein Gasgemisch aus Umgebungsluft und/oder Druckluft und/oder einem zumindest teilweise zurückgeatmeten Atemgas ist.

11. Beatmungsgerät (200) nach Anspruch 6 oder 7,
    wobei die Steuereinheit (214) ausgebildet ist, um das Rückatemvolumen anhand von Messdaten zumindest eines Flusssensors (204, 207, 211) zu berechnen.

**Claims**

1. A ventilator (200), comprising a gas valve (203) and a control unit (214) which is designed to carry out a method (100) for regulating a gas flow (146) of at least one first gas to be admixed with at least one second gas, wherein the method (100) comprises at least one method step (140) of controlling (145) the gas valve (203), wherein at least one control variable for controlling (145) the gas valve (203) is determined from at least one correction regulator component (143) and at least one feed-forward component (144), wherein the input variable of the feed-forward component (144) is a predicted gas flow setpoint value (133, 134) of the first gas, wherein the method (100) further comprises:

   a first method step (110) of the total flow prediction, wherein, starting from a set pressure value (111) and a ventilation situation (112), a predicted total flow setpoint value (116) of the gas mixture of the at least two gases is determined;
   a second method step (120) for flow scaling, wherein, from the predicted total flow setpoint value (116) alone or from the predicted total flow setpoint value (116) together with the predicted gas flow setpoint value (133), a scaled predicted total flow setpoint value (127) is calculated;
   a third method step (130) for determining a scaled predicted gas flow setpoint value (134) from the scaled predicted total flow setpoint value (127);
   wherein the input variable for the feed-forward component (144) for determining the control variable for controlling (145) the gas valve (203) is the scaled predicted gas flow setpoint value (134).

2. The ventilator (200) according to claim 1,
   wherein the first method step (110) comprises a patient flow model (114), wherein the set pressure value (111) and the ventilation situation (112) are at least partially incorporated into the patient flow model (114) and wherein the result of the patient flow model (114) and at least partially the ventilation situation (112) are incorporated into a calculation (115) of the predicted total flow setpoint value (116).

3. The ventilator (200) according to claim 2,
   wherein, in the first method step (110), starting values for the patient flow model (114) are determined from the ventilation situation (112) using a patient model, in particular in the form of an RC equivalent model (113).

4. The ventilator (200) according to any one of the preceding claims,
   wherein the second method step (120) comprises a scaling (126), wherein separate scaling factors and/or scaling functions are determined for the inspiration and expiration and, depending on inspiration or expiration, a switch (125) is used to switch between the scaling factors and/or scaling functions.

5. The ventilator (200) according to claim 4,
   wherein, to determine the scaling factors and/or the scaling functions, at least one comparison between a provided inspiration or expiration volume and an actually applied inspiration or expiration volume is taken into account.

6. The ventilator (200) according to any one of the preceding claims,
   wherein the third method step (130) comprises determining an average concentration mcO2% of the first gas in the second gas, wherein, when determining the average concentration mcO2% of the first gas, a rebreathing volume is included.

7. The ventilator (200) according to any one of the preceding claims,
wherein the input variable for the feed-forward component (144) for determining the control variable for controlling (145) the gas valve (203) is the scaled predicted gas flow setpoint value (134) with a rebreathing volume being taken into account.

8. The ventilator (200) according to any one of the preceding claims,
wherein an input variable for the correction regulator component (143) comprises at least one parameter which describes a deviation of an actual gas flow value (147) determined by at least one flow sensor (204) from a predetermined gas flow setpoint value (141).

9. The ventilator (200) according to claim 8,
wherein the correction regulator component (143) of the control variable for controlling (145) the gas valve (203) becomes zero when the actual gas flow value (147) corresponds to the setpoint gas flow value (141).

10. The ventilator (200) according to any one of the preceding claims,
wherein the first gas is oxygen and the second gas is ambient air or compressed air or a gas mixture consisting of ambient air and/or compressed air and/or an at least partially rebreathed respiratory gas.

11. The ventilator (200) according to claim 6 or 7,
wherein the control unit (214) is designed to calculate the rebreathing volume using measurement data from at least one flow sensor (204, 207, 211).

## Revendications

1. Appareil respiratoire (200), comportant une soupape de gaz (203) et une unité de commande (214), laquelle est conçue pour mettre en œuvre un procédé (100) destiné à la régulation d'un flux de gaz (146) d'au moins un premier gaz destiné à être mélangé à un deuxième gaz, dans lequel le procédé (100) comporte au moins une étape de procédé (140) d'une commande (145) de la soupape de gaz (203), dans lequel au moins une grandeur de réglage pour la commande (145) de la soupape de gaz (203) est déterminée à partir d'au moins une part de régulateur de correction (143) et d'au moins une part d'anticipation (144), dans lequel la grandeur d'entrée de la part d'anticipation (144) est une valeur de consigne de flux de gaz prévue (133, 134) du premier gaz, dans lequel le procédé (100) comporte en outre :

   une première étape de procédé (110) de la prévision de flux global, dans lequel une valeur de consigne de flux global prévue (116) du mélange de gaz des au moins deux gaz est déterminée à partir d'une valeur de pression réglée (111) et d'une situation de ventilation (112) ;
   une deuxième étape de procédé (120) pour la mise à l'échelle des flux, dans lequel une valeur de consigne de flux global prévue mise à l'échelle (127) est calculée à partir de la valeur de consigne de flux global prévue (116) seule ou à partir de la valeur de consigne de flux global prévue (116) ensemble avec la valeur de consigne de flux de gaz prévue (133) ;
   une troisième étape de procédé (130) pour la détermination d'une valeur de consigne de flux de gaz prévue mise à l'échelle (134) à partir de la valeur de consigne de flux global prévue mise à l'échelle (127) ;
   dans lequel la grandeur d'entrée pour la part d'anticipation (144) pour la détermination de la grandeur de réglage pour la commande (145) de la soupape de gaz (203) est la valeur de consigne de flux de gaz prévue mise à l'échelle (134).

2. Appareil respiratoire (200) selon la revendication 1,
dans lequel la première étape de procédé (110) comporte un modèle de flux de patient (114), dans lequel la valeur de pression réglée (111) et la situation de ventilation (112) sont au moins partiellement intégrées dans le modèle de flux de patient (114) et dans lequel le résultat du modèle de flux de patient (114) ainsi qu'au moins partiellement la situation de ventilation (112) sont intégrés dans un calcul (115) de la valeur de consigne de flux global prévue (116).

3. Appareil respiratoire (200) selon la revendication 2,
dans lequel, dans la première étape de procédé (110), des valeurs de sortie sont déterminées pour le modèle de flux de patient (114) à partir de la situation de ventilation (112) via un modèle de patient, en particulier sous la forme d'un modèle de remplacement RC (113).

4. Appareil respiratoire (200) selon l'une des revendications précédentes,
dans lequel la deuxième étape de procédé (120) comporte une mise à l'échelle (126), dans lequel des facteurs d'échelle et/ou des fonctions de mise à l'échelle séparés sont déterminés pour l'inspiration et l'expiration et une commutation est effectuée entre les fonctions de mise à l'échelle et/ou les facteurs d'échelle par le biais d'un commutateur (125) selon l'inspiration ou l'expiration.

5. Appareil respiratoire (200) selon la revendication 4,
dans lequel au moins une comparaison entre un volume d'inspiration ou d'expiration prévu et un volume d'inspiration ou d'expiration réellement appliqué est prise en compte dans la détermination des facteurs d'échelle et/ou des fonctions de mise à l'échelle.

6. Appareil respiratoire (200) selon l'une des revendications précédentes,
dans lequel la troisième étape de procédé (130) comporte la détermination d'une concentration moyenne mcO2 % du premier gaz dans le deuxième gaz, dans lequel un volume respiratoire de retour est inclus dans la détermination de la concentration moyenne mcO2 % du premier gaz.

7. Appareil respiratoire (200) selon l'une des revendications précédentes,
dans lequel la grandeur d'entrée pour la part d'anticipation (144) pour la détermination de la grandeur de réglage pour la commande (145) de la soupape de gaz (203) est la valeur de consigne de flux de gaz prévue mise à l'échelle (134) avec prise en compte d'un volume respiratoire de retour.

8. Appareil respiratoire (200) selon l'une des revendications précédentes,
dans lequel une grandeur d'entrée pour la part de régulateur de correction (143) comporte au moins un paramètre, lequel décrit un écart d'une valeur réelle de flux de gaz (147) déterminée par au moins un capteur de flux (204) par rapport à une valeur de consigne de flux de gaz prédéfinie (141).

9. Appareil respiratoire (200) selon la revendication 8,
dans lequel la part de régulateur de correction (143) de la grandeur de réglage pour la commande (145) de la soupape de gaz (203) devient nulle lorsque la valeur réelle de flux de gaz (147) correspond à la valeur de consigne du flux de gaz (141).

10. Appareil respiratoire (200) selon l'une des revendications précédentes,
dans lequel le premier gaz est de l'oxygène et le deuxième gaz est de l'air ambiant ou de l'air comprimé ou un mélange gazeux d'air ambiant et/ou d'air comprimé et/ou d'un gaz respiratoire au moins partiellement renvoyé.

11. Appareil respiratoire (200) selon la revendication 6 ou 7,
dans lequel l'unité de commande (214) est conçue pour calculer le volume respiratoire de retour à l'aide de données de mesure d'au moins un capteur de flux (204, 207, 211).

**Figur 1**

**Figur 2**

**Figur 3**

**Figur 4**

**Figur 5**

**Figur 6**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20160287824 A1
- US 10821259 B2
- US 20210361899 A1
- US 6216690 B1